# FASCICULE DE BREVET EUROPEEN

(11) **EP 1 317 922 B1**
(45) Date de publication et mention de la délivrance du brevet: **18.04.2007**
(21) Numéro de dépôt: 02292961.6
(22) Date de dépôt: 02.12.2002
(51) Int. Cl.: A61K 31/047, A61K 31/60, A61P 29/00

(54) **Preparation antipyretique renfermant du xylitol**
Antipyretisches Präparat enhaltend Xylitol
Antipyretic preparation containing xylitol

(30) Priorité: 10.12.2001 FR 0115917
(43) Date de publication de la demande: 11.06.2003
(73) Titulaire: Roquette Frères, 62136 Lestrem (FR)
(72) Inventeur: Wils, Daniel, 59190 Morbecque (FR); Bernard, Roselyne, 62136 Lestrem (FR)
(74) Mandataire: Boulinguiez, Didier

(56) Documents cités:
- GB-A- 1 212 115
- US-A- 5 922 347
- SHIMOHIRA A. TOKYO TORITSU EISEI KENKYUSHO KENYU NEMPO vol. 1971, 1969, pages 83 - 88 & FRENCH TRANSLATION SUBMITTED BY APPLICANT

## Description

La présente invention est relative à l'utilisation du xylitol en combinaison avec l'aspirine pour la préparation d'un médicament antipyrétique.

Plus particulièrement, l'invention concerne l'utilisation d'une préparation antipyrétique constituée d'un antipyrétique et d'une quantité synergique de xylitol, cette préparation étant destinée à être administrée chez l'homme ou l'animal par tout moyen autre que la voie orale.

Au sens de l'invention, on entend par « antipyrétique », ou « agent antipyrétique », un composé ayant la propriété d'abaisser la température corporelle chez l'homme ou chez l'animal, quand celle-ci se trouve à un niveau anormalement élevé par rapport aux valeurs physiologiques pour l'homme ou l'animal.

Au sens de l'invention, on entend également par « quantité efficace » de xylitol, une quantité de xylitol qui, agissant en synergie avec un antipyrétique, permet d'optimiser l'abaissement de la température corporelle.

De nombreuses substances sont connues depuis longtemps pour leurs effets pyrogènes au sens large, chez l'homme et les animaux. Il s'agit du soufre, du lait, de certaines gommes, de certaines hormones stéroïdiennes, du dinitro-2,4 phénol, de la tétra-hydro-β-naphtylamine et du carmin.

Les métabolites synthétisés par certains microorganismes sont connus également de longue date pour leurs propriétés pyrogènes, tels les endotoxines de bactéries Gram négatif ou l'ARN de *Candida utilis*.

En réponse aux élévations de température constatées chez l'homme ou l'animal exposés à ces agents, sont utilisés des principes actifs particuliers abaissant la température corporelle, principes actifs appelés antipyrétiques.

Pour ramener la température corporelle à sa valeur physiologique (par exemple 37°C pour l'homme), l'organisme augmente ses pertes de chaleur, par des mécanismes de type vasodilatation périphérique, hypersudation et inhibition de la synthèse des prostaglandines au niveau des centres thermorégulateurs.

Pour abaisser la température corporelle, sont classiquement utilisés des analgésiques antipyrétiques à action anti-inflammatoire notable comme l'aspirine et ses dérivés, et des analgésiques antipyrétiques pratiquement dépourvus de propriétés anti-inflammatoires comme les dérivés du paraminophénol (tels que le paracétamol), les dérivés de la pyrazolone et les diverses associations médicamenteuses avec les analgésiques antipyrétiques.

L'homme du métier retrouve aisément les modes d'administration et les posologies de tous ces antipyrétiques dans le dictionnaire VIDAL.

Il faut noter cependant qu'à côté du développement de ces substances à effets antipyrétiques, un certain nombre de travaux intégrant des polyols ont été également entrepris.

GB 1 212 115 divulgue un comprimé contenant de l'acétylsalicylique de magnésium. Ce document indique que l'administration d'une dose de 100 mg/kg/jour d'acide acétylsalicylique est néfaste pour l'organisme car elle peut entraîner une irritation de la muqueuse digestive, des ulcères de l'estomac ou des hémorragies nasales. Afin de prévenir de tels effets secondaires, GB 1 212 115 recommande de substituer l'acide acétylsalicylique par l'acétylsalicylique de magnésium stabilisé dans une solution d'alcool anhydre.

En effet, outre leurs propriétés comme agents de texture à saveur sucrée et leur remarquable stabilité chimique, ce sont surtout leurs propriétés de stabilité bactériologique et leur non fermentescibilité qui ont conduit à l'utilisation des polyols non seulement dans le domaine alimentaire, mais également en thérapie humaine.

C'est ainsi que dans le cas particulier du xylitol, outre son usage comme édulcorant chez les diabétiques et comme agent acariogène, il a été aussi décrit dans un certain nombre d'applications thérapeutiques comme l'illustrent les enseignements des brevets suivants :
- dans le brevet EP 150.053, le xylitol est incorporé dans des préparations aqueuses hypocaloriques, faiblement osmotiques délivrées en perfusion à des patients dans un état extrêmement grave, souffrant de stress ou de traumatisme sévère, de maladie rénale ou placés sous ventilation. Le xylitol apporte la seule source d'énergie de ces préparations.
- dans le brevet US 5.719.196, le xylitol est incorporé dans des préparations orales destinées chez l'homme à traiter des infections de type otite aiguë, des infections respiratoires graves, la bronchite aiguë, la sinusite et la conjonctivite. Le xylitol, sous forme solide, est administré à raison de 6 à 10 g par jour, et il est décrit comme inhibiteur de croissances des microorganismes pathogènes responsables de ces diverses infections.

Le xylitol est enfin utilisé dans d'autres nombreuses applications médicales telles la prévention de la suppression adénocorticale durant la thérapie stéroïdienne, l'amélioration de l'hémolyse induite par certaines drogues, la réduction des dysfonctionnements du foie et de la bile... comme l'illustre l'article de K.K. MÂKINEN dans *Medical Hypotheses* (2000), 54-4, pp 603-613.

A la connaissance de la société Demanderesse, cependant, seuls les essais entrepris par A. SHIMOHIRA rapportés dans *TOKYO TORITSU EISEI KENKYUSHO KENKYU NEMPO* en 1969 (volume 1971 pp 83-88) traitent quant à eux d'effets d'abaissement de température corporelle en relation avec l'administration de xylitol par voie intraveineuse.

Il s'agissait de déterminer la diminution de la température corporelle d'un animal de laboratoire, en l'occurrence un lapin sain soumis à l'action de diverses molécules administrées par voies intraveineuses.

Le xylitol a ainsi été testé à côté des molécules comme l'éthyl éphédrine et la méthyl éphédrine.

Les résultats indiquent que le xylitol, tout comme la méthyl éphédrine, abaisse la température corporelle de l'animal de laboratoire.

Cette étude, réalisée chez des animaux en bonne santé, enseigne que le xylitol active les phénomènes dits de thermolyse qui conduisent à l'abaissement de la température corporelle chez l'animal.

Cependant, l'enseignement que l'homme du métier peut tirer de cette observation est surtout le fait que le xylitol, chez l'animal de laboratoire, et *a fortiori* chez l'homme, présente un effet d'abaissement de température chez l'individu sain.

US 5, 922, 34 divulgue l'utilisation d'un chewing-gum comprenant du xylitol et de l'acide acétylsalicylique afin de diminuer la température corporelle. L'acide acétylsalicylique est donc administré par voie orale.

La société Demanderesse a cependant eu le mérite de reprendre les travaux sur le xylitol dans le but de mettre au point des préparations susceptibles de diminuer la température corporelle, non pas pour des individus sains, mais dans des cas de fièvre induite lors d'un processus infectieux.

En effet, il est connu de l'homme du métier que les mécanismes d'abaissement de température d'un individu malade diffèrent fondamentalement de ceux qui régissent l'abaissement de la température corporelle d'un individu sain.

A la connaissance de la société Demanderesse, il n'est nulle part décrit ou suggéré dans l'état de la technique que le xylitol puisse avoir un même effet dans ces deux problématiques physiologiques si dissemblables.

Après de nombreuses investigations, la société Demanderesse a ainsi montré que le xylitol pouvait être administré, par une voie autre que la voie orale, avec un antipyrétique lors d'une élévation de température résultant d'une stimulation du système immunitaire, d'une infection, d'un processus inflammatoire, qui sont des phénomènes mettant en jeu d'autres mécanismes physiologiques que la thermogenèse.

Plus particulièrement, la société Demanderesse a réussi à élaborer, au prix de nombreuses recherches, des préparations antipyrétiques destinées à être administrées par tout moyen autre que la voie orale, préparations constituées de xylitol ou d'un antipyrétique et d'une quantité synergique de xylitol.

La société Demanderesse dispose en effet d'une unité de production de xylitol apyrogène dont les applications visées sont celles évoquées ci-dessus.

La société Demanderesse a donc entrepris, sur des animaux de laboratoire présentant une hyperthermie, des essais comparatifs visant à étudier les effets d'abaissement de la température corporelle d'un médicament connu pour ses propriétés antipyrétiques en regard du xylitol pris seul ou en combinaison.

Contre toute attente, étant donné la nature complexe et multifactorielle des mécanismes physiologiques développés dans l'organisme pour abaisser la température corporelle d'un animal en bonne santé, ou au contraire d'un animal en hyperthermie, la société Demanderesse a ainsi montré qu'il était possible, grâce au xylitol, non seulement d'abaisser la température corporelle du lapin malade de façon remarquable, mais aussi, de potentialiser grâce au xylitol les effets d'abaissement de température d'antipyrétiques classiques.

L'effet supplémentaire apporté par le xylitol n'est donc pas un effet additionnel de deux agents antipyrétiques différents, mais bien d'une combinaison d'effets, l'écart de température constaté étant bien supérieur à l'écart de température provoqué par chacun des deux composants de la préparation administrée pris séparément, comme il sera exemplifié ci-après.

La société Demanderesse a donc trouvé que cet effet peut être apporté par la confection d'une préparation antipyrétique destinée à être administrée par tout moyen autre que la voie orale, caractérisée en ce qu'elle comprend du xylitol.

De manière surprenante et inattendue, la société Demanderesse a également constaté que cet effet antipyrétique pouvait être augmenté par une préparation constituée d'un antipyrétique et d'une quantité synergique de xylitol.

La présente invention a ainsi pour objet l'utilisation du xylitol en combinaison avec l'aspirine pour la préparation d'un médicament antipyrétique pour l'administration par voie intraveineuse, intramusculaire, intrapéritonéale ou rectale, destiné à abaisser la température corporelle dans le traitement d'infections parasitaires, virales ou bactériennes, caractérisée en ce que l'administration journalière du médicament comprend une quantité de xylitol comprise entre 0,5 et 15 g et une quantité d'aspirine comprise entre 2 et 100 mg, ces quantités étant exprimées par kg de poids corporel.

Les essais réalisés chez le lapin, animal de référence choisi comme modèle pour apprécier les hyperthermies provoquées par certaines molécules, ont permis d'établir les proportions de xylitol et d'agents antipyrétiques permettant de mettre en évidence l'effet du xylitol seul, ou l'effet de synergie lorsque associé avec un agent antipyrétique.

En effet, le test des pyrogènes chez le lapin est le test décrit dans la pharmacopée européenne pour vérifier *in vivo* l'apyrogénicité de certaines molécules destinées à la voie parentérale.

C'est ainsi que la société Demanderesse, productrice de molécules destinées à la voie parentérale, a donc utilisé ce test qu'elle maîtrise parfaitement dans les conditions décrites dans la pharmacopée européenne pour déterminer les effets d'abaissement de la température corporelle du xylitol seul, et de xylitol combiné avec un antipyrétique.

La société Demanderesse a ainsi trouvé que dans la préparation antipyrétique destinée à être administrée par une voie autre que la voie orale la teneur en xylitol est comprise entre 0,5 et 15 g, ces teneurs s'entendant en quantité administrable par kg de poids corporel et par jour.

De la même manière, la société Demanderesse a montré que cette quantité de xylitol, lorsqu'elle est combinée avec une quantité d'un antipyrétique comprise entre 2 et 100 mg / kg de poids corporel, conduisait à augmenter de manière significative l'effet d'abaissement de la température corporelle de l'animal de laboratoire en hyperthermie, abaissement de la température amené par l'antipyrétique seul.

Comme substance antipyrétique, il a été choisi un composé de la famille des analgésiques antipyrétiques à action anti-inflammatoire de la famille de l'aspirine, en l'occurrence l'ASPEGIC^{®}.

Les effets remarquables d'abaissement de température corporelle constatés destinent ainsi les préparations injectables aux applications pharmaceutiques, où le xylitol peut alors être utilisé comme médicament à effet antipyrétique, par exemple en association avec un autre médicament de la famille des analgésiques antipyrétiques à action anti-inflammatoire ou non.

Il est également possible d'utiliser la préparation dans le traitement des infections choisies dans le groupe des infections parasitaires, virales et bactériennes, mais aussi dans le traitement des coups de chaleur, des brûlures, des intoxications par produit chimique, ou de tout dérèglement de la fonction de régulation de la température corporelle.

D'autres caractéristiques et avantages de l'invention apparaîtront à la lecture de l'exemple qui suit.

### Exemple 1

L'expérience est menée avec des lots d'animaux de laboratoire, en l'occurrence ici le lapin.

Les solutions à injecter sont préparées dans de l'eau stérile (Eau Pour Préparation Injectable ou EPPI - AGUETTANT).

Les flacons d'endotoxines (Endotoxine Standard de Contrôle ou CSE - ENDOSAFE) renferment 5 ml d'endotoxines à 30 EU/ml avec 120 ml d'EPPI.

La contamination est calculée de manière à observer un choc pyrogène chez le lapin. Celle-ci est réalisée par injection intraveineuse d'endotoxines, à raison de 1,2 EU/ml, soit 12 EU/kg.

L'agent antipyrétique, en l'occurrence de l'aspirine, est préparé de façon extemporanée. 6,6 ml sont ajoutés aux 125 ml d'endotoxines de manière à obtenir une dose équivalant à 100 mg/kg. L'aspirine choisie est de l'ASPEGIC^{®} (acétylsalicylate de DL-lysine) injectable, fabriquée par les laboratoires SYNTHELABO (LE PLESSIS ROBINSON), conditionnée à raison de 6 flacons de 2 g de poudre et 6 ampoules de 5 ml d'EPPI pour dissolution extemporanée.

Le xylitol est un lot de XYLISORB^{®} apyrogène fabriqué par la société Demanderesse. Du dextrose monohydrate apyrogène (commercialisé sous la marque LYCADEX^{®} PF par la société Demanderesse) est utilisé comme témoin.

Les lapins sont injectés avec une solution de xylitol à 150 g/l avec de l'EPPI, contaminée ou non par les endotoxines. 5 expériences sont réalisées à raison de 3 lapins par lot. Le tableau I suivant présente la composition des solutions injectées aux 5 lots de trois lapins.

**Tableau I**

| N° du lot | Essais | Produit injecté | EPPI contaminée | Aspirine |
|---|---|---|---|---|
| 1 | Témoin négatif | LYCADEX^{®} PF | NON | NON |
| 2 | Témoin positif | LYCADEX^{®} PF | OUI | NON |
| 3 | Témoin Aspirine | LYCADEX^{®} PF | OUI | OUI |
| 4 | Xylitol seul | XYLISORB apyrogène | OUI | NON |
| 5 | Xylitol + Aspirine | XYLISORB apyrogène | OUI | OUI |

Les résultats des mesures des écarts de températures moyens effectuées sur les 5 lots de lapins sont donnés dans le tableau II suivant.

**Tableau II**

| | Elévation de Température (°C) | Ecart par rapport au témoin positif de contamination (%) |
|---|---|---|
| LOT 1 | 0,35 | - |
| LOT 2 | 2,95 | O |
| LOT 3 | 1,57 | 46 |
| LOT 4 | 2,73 | 7,5 |
| LOT 5 | 0,82 | 72 |

Une différence significative est établie quand l'écart est supérieur à 5 %.

Il s'ensuit que l'injection de xylitol présente déjà un effet significatif, puisque l'on constate un abaissement de température de 7,5 %.

Ensuite, l'effet remarquable constaté est que, combiné avec l'aspirine, le xylitol permet d'abaisser ici de 72 % l'élévation de température associée à l'injection d'endotoxines, alors que l'aspirine seule, dans les mêmes conditions opératoires, n'abaisse l'élévation de température que de 46 %.

Il existe donc bien une synergie entre le xylitol (ici à 1,5 g/kg) et l'ASPEGIC^{®} (ici à 100 mg/kg) injectés par voie intraveineuse, pour activer la thermolyse chez le lapin ayant reçu une dose d'endotoxines égale à 12 EU/kg.

Cette propriété du xylitol pourrait être dès lors exploitée dans le domaine des excipients de médicaments à visée antipyrétique.

## Revendications

1. Utilisation du xylitol en combinaison avec l'aspirine pour la préparation d'un médicament antipyrétique pour l'administration par voie intraveineuse, intramusculaire, intrapéritonéale ou rectale, destiné à abaisser la température corporelle dans le traitement d'infections parasitaires, virales ou bactériennes, **caractérisée en ce que** l'administration journalière du médicament comprend une quantité de xylitol comprise entre 0,5 et 15 g et une quantité d'aspirine comprise entre 2 et 100 mg, ces quantités étant exprimées par kg de poids corporel.

2. Utilisation selon la revendication 1, **caractérisée en ce que** l'administration du médicament antipyrétique est en association avec un autre médicament de la famille des analgésiques antipyrétiques à action anti-inflammatoire.

3. Utilisation selon la revendication 1 ou 2 dans le traitement des coups de chaleur, des brûlures, des intoxications par produits chimiques ou de tout dérèglement de la fonction de régulation de la température corporelle.

## Claims

1. Use of xylitol in combination with aspirin for the preparation of an antipyretic medicinal product for intravenous, intramuscular, intraperitoneal or rectal administration, intended to decrease body temperature in the treatment of parasitic, viral or bacterial infections, **characterized in that** the daily administration of the medicinal product comprises an amount of xylitol of between 0.5 and 15 g and an amount of aspirin of between 2 and 100 mg, these amounts being expressed per kg of bodyweight.

2. Use according to Claim 1, **characterized in that** the administration of the antipyretic medicinal product is in combination with another medicinal product of the family of antipyretic analgesics with an anti-inflammatory action.

3. Use according to Claim 1 or 2 in the treatment of heatstroke, burns, poisoning with chemical products or any disturbance of the function of body temperature regulation.

## Patentansprüche

1. Verwendung von Xylitol in Kombination mit Aspirin zur Herstellung eines antipyretischen Medikaments zur Verabreichung auf intravenösem, intramuskulärem intraperitonealem oder rektalem Weg, mit dem Ziel, die Körpertemperatur bei der Behandlung von parasitären, viralen oder bakteriellen Infektionen zu senken, **dadurch gekennzeichnet, dass** die tägliche Verabreichung des Medikaments eine Menge von Xylitol von 0,5 bis 15 g und eine Menge von Aspirin von 2 bis 100 mg umfasst, wobei die Mengen pro kg Körpergewicht angegeben sind.

2. Verwendung nach Anspruch 1, **dadurch gekennzeichnet, dass** die Verabreichung des antipyretischen Medikaments in Verbindung mit einem anderem Medikament der Familie der antipyretischen Analgetika mit anti-inflammatorischer Wirkung erfolgt.

3. Verwendung nach Anspruch 1 oder 2 bei der Behandlung von Hitzeschlag, Verbrennungen, Vergiftungen durch chemische Produkte oder bei der Behandlung von einer vollständigen Fehlfunktion der Körpertemperatur-Regulation.
